# FASCICULE DE BREVET EUROPEEN

(11) **EP 0 579 559 B1**
(45) Date de publication et mention de la délivrance du brevet: **08.03.1995**
(21) Numéro de dépôt: 93420263.1
(22) Date de dépôt: 22.06.1993
(51) Int. Cl.: G01F 25/00, A61M 1/16

(54) **Procédé d'étalonnage d'un couple de capteurs placés dans un circuit de dialyse**
Verfahren zur Eichung eines Paares von Sensoren in einem Dialysatkreislauf
Calibration method for a couple of transducers positioned in a dialysis circuit

(30) Priorité: 30.06.1992 FR 9208287
(43) Date de publication de la demande: 19.01.1994
(73) Titulaire: HOSPAL INDUSTRIE, F-69883 Meyzieu Cédex (FR)
(72) Inventeur: Bene, Bernard, F-69540 Irigny (FR); Chevallet, Jacques, F-69360 Serezin du Rhône (FR)

(56) Documents cités:
- EP-A- 0 298 587
- FR-A- 2 651 037

## Description

La présente invention concerne le domaine technique général de la mesure de grandeurs physiques d'un liquide de dialyse circulant dans un hémodialyseur d'un rein artificiel et elle vise, plus précisément, l'étalonnage des capteurs utilisés pour mesurer les grandeurs chimiques ou physiques du liquide de dialyse.

L'invention trouve une application particulièrement avantageuse pour la mesure du débit du liquide de dialyse circulant dans l'hémodialyseur d'un rein artificiel.

Dans l'application préférée citée ci-dessus, un rein artificiel comporte, d'une manière classique, un hémodialyseur à deux compartiments séparés par une membrane, dont l'un des compartiments est relié à un circuit de liquide de dialyse, tandis que l'autre compartiment est relié à un patient, par l'intermédiaire d'un circuit extracorporel de sang. Le circuit de liquide de dialyse est pourvu, en entrée et en sortie de l'hémodialyseur, de débitmètres générant une impulsion électrique au passage d'une fraction déterminée de liquide de dialyse.

Au cours d'une séance de dialyse, le liquide en excès présent dans le sang, ultrafiltre à travers une membrane, en raison du gradient de pression exercé de part et d'autre de la membrane. Préalablement à une telle séance, il convient de procéder à une phase d'étalonnage des débitmètres pour laquelle l'hémodialyseur est court-circuité, de sorte qu'un débit identique de liquide de dialyse circule dans les deux débitmètres. Cette phase d'étalonnage consiste à mesurer les fréquences d'étalonnage de chaque débitmètre en comptant le nombre d'impulsions pendant un temps de durée choisie, de manière à définir le facteur de réponse, c'est-à-dire la relation existant entre une fréquence de mesure et un débit donné de liquide de dialyse. Une telle phase d'étalonnage permet de corriger, en partie, les erreurs intrinsèques des débitmètres et même d'utiliser des débitmètres de type différent.

Il convient de considérer que l'étalonnage des débitmètres, qui résulte de mesures expérimentales, est entaché d'une certaine erreur affectant systématiquement les résultats de mesures ultérieures. Il apparaît donc indispensable de réduire au maximum les erreurs accidentelles de mesure, susceptibles d'apparaître au cours de la phase d'étalonnage, de manière à obtenir une précision de mesure maximale pour la détermination de l'ultrafiltrat retiré du sang du patient.

Pour tenter de réduire l'importance des erreurs de mesure, telles que les artefacts, la technique antérieure propose, généralement, de procéder à une phase d'étalonnage sur une durée assez longue.

Par ailleurs, la demande de brevet **EP A 0 298 587** propose un procédé tentant de limiter les erreurs de mesure de débits, notamment au cours d'une phase d'étalonnage de débitmètres équipant un hémodialyseur. Ce document propose de monter en série, sur le circuit de liquide de dialyse, un couple de débitmètres en amont de l'hémodialyseur et un autre couple, en aval de l'hémodialyseur. Au cours de la phase d'étalonnage, l'hémodialyseur est court-circuité, de sorte que les quatre débitmètres se retrouvent placés en série. Les fréquences des débitmètres sont mesurées et l'étalonnage est considéré valable si les écarts entre les fréquences mesurées sont compris dans une plage déterminée de valeurs.

Le principal inconvénient du procédé décrit ci-dessus réside dans le fait que la précision des mesures dépend directement du temps d'acquisition pendant lequel les impulsions sont comptées. Aussi, pour atteindre une précision élevée, il convient d'augmenter, de manière prohibitive, le temps d'acquisition des mesures.

Par ailleurs, il s'avère, en pratique, que les débitmètres présentent une dérive de mesure au cours d'une séance de dialyse dont la durée atteint, généralement, quatre heures. Il devient alors nécessaire de procéder à des étalonnages en cours de séance de dialyse pour éliminer cette erreur, ce qui diminue l'efficacité de la dialyse, en raison de la longue durée de la phase d'étalonnage.

De plus, le doublement des débitmètres augmente le coût de mise en oeuvre d'un tel procédé.

Pour résoudre les inconvénients énoncés ci-dessus, le brevet **FR-A-2 651 037** a proposé un procédé d'étalonnage permettant d'obtenir une précision de mesure élevée, indépendante du temps d'étalonnage. Le temps d'étalonnage, nécessaire à la mise en oeuvre du procédé, peut être limité à une durée minimum, sans toutefois affecter la précision de mesure, puisque les erreurs susceptibles d'intervenir pendant ce temps d'étalonnage, se trouvent éliminées par le procédé d'étalonnage proposé. Cette phase d'étalonnage des débitmètres, pendant laquelle l'hémodialyseur est mis en court circuit, peut être effectuée au cours d'une séance de dialyse, en raison de la courte durée nécessaire pour procéder à cet étalonnage.

Bien entendu, si le nombre d'étalonnages devient important en vue d'augmenter la précision des mesures, la somme des temps nécessaires aux étalonnages successifs aboutit à une durée d'étalonnage non négligeable. Cette durée globale de l'étalonnage, pendant laquelle l'hémodialyseur est mis en court-circuit, conduit à une diminution de l'efficacité de la séance de dialyse. Un compromis doit donc être effectué entre l'efficacité de la séance de dialyse et la précision des mesures, sans toutefois négliger la sécurité qui nécessite de déceler, au plus tôt, l'apparition d'un incident sur les débitmètres.

Il apparaît donc le besoin de disposer d'une méthode d'étalonnage des débitmètres permettant d'obtenir une précision élevée sur les mesures, tout au long de la séance de dialyse et une efficacité optimale pour cette séance en s'affranchissant de la mise en court-circuit de l'hémodialyseur pendant la séance.

L'objet de la présente invention vise, justement, un procédé d'étalonnage de capteurs, destiné à satisfaire le besoin énoncé ci-dessus.

L'objet de l'invention vise, également, à proposer un procédé d'étalonnage offrant une grande sécurité pour déceler d'éventuels défauts susceptibles d'intervenir sur les capteurs au cours d'une séance de dialyse.

Pour atteindre les objectifs énoncés ci-dessus, le procédé, qui assure l'étalonnage d'un couple de capteurs, dont l'un est monté à l'entrée et l'autre à la sortie d'un circuit de dialyse destiné à être relié à un hémodialyseur, consiste :
- à effectuer une phase initiale d'étalonnage des capteurs pendant laquelle l'hémodialyseur est mis en court-circuit, de manière à déterminer le facteur de réponse de chaque capteur et à définir un coefficient initial d'étalonnage permettant d'obtenir une valeur identique de mesure pour chaque capteur,
- et à réaliser au moins une phase de correction du coefficient initial d'étalonnage, au cours d'une séance de fonctionnement de l'hémodialyseur.

Selon l'invention, le procédé consiste :
- à réaliser au moins une phase de détermination d'un coefficient auxiliaire d'étalonnage pour le capteur d'entrée consistant :
   . à assurer la mise en série, avec le capteur d'entrée, d'un capteur auxiliaire de même nature que les capteurs d'entrée et de sortie,
   . à définir les facteurs de réponse pour les capteurs d'entrée et auxiliaire,
   . et à définir le coefficient auxiliaire d'étalonnage pour le capteur d'entrée, permettant d'obtenir une même valeur de mesure pour les capteurs d'entrée et auxiliaire,
- à réaliser au moins une phase de détermination d'un coefficient auxiliaire d'étalonnage pour le capteur de sortie, consistant :
   . à assurer la mise en série du capteur de sortie avec le capteur auxiliaire,
   . à définir les facteurs de réponse pour les capteurs de sortie et auxiliaire,
   . et à définir le coefficient d'étalonnage auxiliaire pour le capteur de sortie, permettant d'obtenir une même valeur de mesure pour les capteurs de sortie et auxiliaire,
- et à déterminer, à partir du ou des coefficients auxiliaires d'étalonnage des capteurs, un nouveau coefficient d'étalonnage pour les deux capteurs.

Diverses autres caractéristiques ressortent de la description faite ci-dessous en référence aux dessins annexés qui montrent, à titre d'exemples non limitatifs, des formes de réalisation de l'objet de l'invention.

La **fig. 1** est un schéma d'un rein artificiel permettant la mise en oeuvre du procédé d'étalonnage selon l'invention.

Les **fig. 2 à 4** sont des schémas, identiques à celui de la **fig. 1** et montrant le trajet du liquide de dialyse, respectivement, lors d'une phase d'étalonnage des capteurs d'entrée et de sortie, du capteur d'entrée et du capteur de sortie.

La **fig. 5** montre un tableau permettant d'expliciter le procédé d'étalonnage selon l'invention.

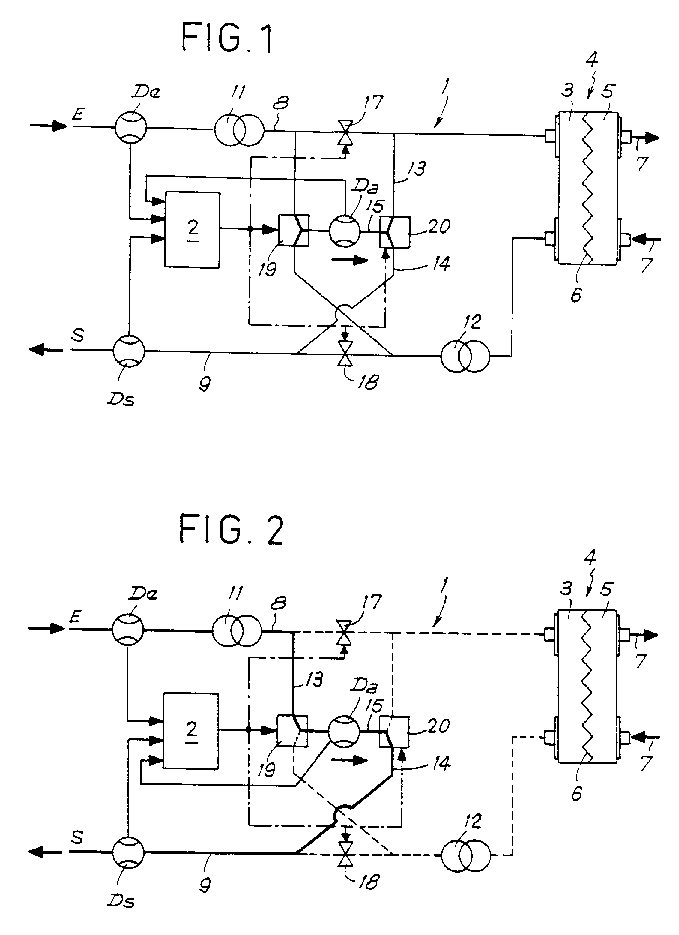

Le rein, représenté sur la **fig. 1** est adapté pour assurer l'étalonnage d'un couple de capteurs **De**, **Ds**, destinés à assurer la mesure de grandeurs physiques ou chimiques d'un liquide de dialyse circulant dans un circuit **1**. Dans l'exemple illustré, les capteurs **De**, **Ds** constituent des débitmètres au sens général, reliés à un dispositif de mesure et de commande **2** dont la fonction apparaîtra plus précisément dans la suite de la description.

Le circuit **1** est relié au premier compartiment **3** d'un hémodialyseur **4** qui comporte, de façon classique, un second compartiment **5**, séparé du premier par une membrane **6** et relié à un patient par l'intermédiaire d'un circuit extracorporel de sang **7**. Le circuit **1** comporte une canalisation d'entrée ou amont **8** équipée du débitmètre d'entrée **De** et reliée, d'une part, par son entrée **E** à une source de liquide de dialyse (non représentée) et, d'autre part, à une entrée du compartiment **3**. Le circuit **1** comporte, également, une canalisation de sortie ou aval **9** équipée du débitmètre de sortie **Ds** et reliée, d'une part, à une sortie du compartiment **3** et, d'autre part, par sa sortie **S**, à des moyens d'évacuation ou de recyclage non représentés. Dans l'exemple illustré, les canalisations d'entrée **8** et de sortie **9** sont pourvues de moyens pour déplacer le liquide de dialyse, tels que respectivement des pompes **11**, **12**.

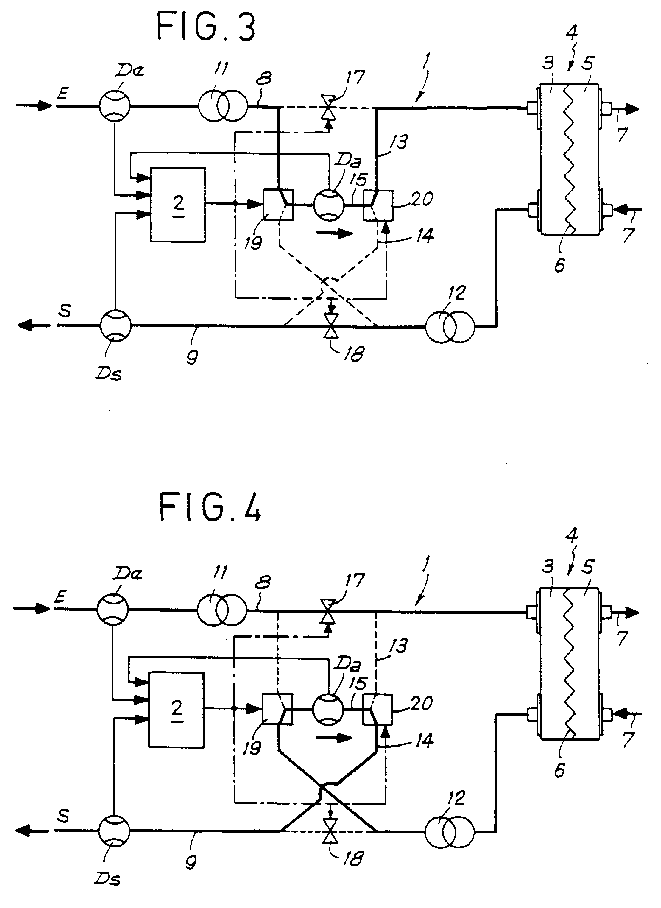

Afin de mettre en oeuvre le procédé d'étalonnage selon l'invention, le circuit de dialyse 1 comporte une branche amont **13** de dérivation à la canalisation d'entrée **8** et une branche aval **14** de dérivation à la canalisation de sortie **9**. Les branches de dérivation **13**, **14** comportent une portion commune **15** dans laquelle est monté un capteur auxiliaire **Da**, tel qu'un débitmètre dans l'exemple illustré, relié au dispositif **2**. Le circuit **1** est pourvu de moyens d'obturation permettant la circulation du liquide de dialyse, soit à travers les trois débitmètres **De**, **Da**, **Ds** montés en série, tandis que l'hémodialyseur est placé en court-circuit (**fig. 2**), soit à travers la branche amont **13** (**fig. 3**), soit à travers la branche aval **14** (**fig.4**).

A titre d'exemple, les moyens d'obturation sont formés par une vanne d'arrêt **17** placée sur la canalisation amont **8** entre le débitmètre **De** et l'hémodialyseur **4**. Les moyens d'obturation comportent, également, une vanne d'arrêt **18** montée sur la canalisation aval **9**, entre le débitmètre **Ds** et l'hémodialyseur **4**. Les moyens d'obturation comportent aussi des vannes **19**, **20** placées de part et d'autre de la portion commune **15** et reliées aux branches de dérivation **13**, **14** qui se raccordent en aval et en amont, respectivement, des vannes d'arrêt **17**, **18**.

Les moyens d'obturation **17**, **18**, **19** et **20** sont pilotés, de préférence, par le dispositif de mesure et de commande **2** qui comporte des moyens, pour la mise en oeuvre du procédé selon l'invention, d'étalonnage des capteurs d'entrée **De** et de sortie **Ds**.

Préalablement à une séance de dialyse, une phase initiale d'étalonnage des débitmètres **De**, **Ds** est effectuée, de manière à obtenir une valeur identique de mesure pour chaque capteur, lors d'une circulation dans ces derniers d'une même valeur de débit du liquide de dialyse. Pour réaliser cet étalonnage initial (**fig. 2**), les vannes **17** et **18** sont fermées, tandis que les vannes **19** et **20** sont commandées pour permettre une circulation du liquide de dialyse à travers une partie de la canalisation amont **8**, une partie de la branche de dérivation **13**, la portion commune **15**, une partie de la branche de dérivation **14** et une partie de la canalisation aval **9**. Les capteurs **De**, **Da**, **Ds** sont ainsi placés en série, tandis que l'hémodialyseur **4** est court-circuité par rapport au circuit **1**. Pendant cette phase d'étalonnage, le débit de liquide, circulant dans les trois débitmètres, est rigoureusement identique dans la mesure où le circuit ainsi formé ne possède aucun point de perte ou de gain de liquide de dialyse.

Le dispositif **2**, qui assure l'acquisition des données provenant des capteurs **De**, **Da**, **Ds**, détermine, respectivement, leurs facteurs de réponse **Feo**, **Fao**, **Fso**, c'est-à-dire la relation existant entre un signal électrique délivré par le capteur et un débit de liquide de dialyse. Le dispositif **2** définit, ensuite, un coefficient auxiliaire **keo** pour le capteur d'entrée **De** et un coefficient auxiliaire **kso** pour le capteur de sortie **Ds**. Chaque coefficient auxiliaire d'étalonnage **keo**, **kso** est formé, par exemple, par le rapport entre, respectivement, les facteurs de réponse **Feo** et **Fao** des capteurs **De**, **Da** et les facteurs de réponse **Fso** et **Fao** des capteurs **Ds**, **Da**, de sorte que **keo** = **Feo**/**Fao** et **kso** = **Fso**/**Fao**. Le dispositif **2** détermine ensuite un coefficient initial d'étalonnage **Ko** qui peut être, par exemple, le rapport entre les coefficients auxiliaires d'étalonnage **keo** et **kso**, tel que **Ko** = **keo**/**kso**.

D'une manière avantageuse, le dispositif **2** détermine, également, un coefficient initial d'étalonnage **Ko'** correspondant au rapport entre les facteurs de réponse **Feo** et **Fso** des capteurs **De**, **Da**, de sorte que **Ko'** = **Feo**/**Fso**. Le dispositif **2** compare les coefficients **Ko** et **Ko'** et si une différence apparaît entre les deux valeurs calculées, le dispositif **2** peut émettre un signal d'avertissement signifiant une fuite au niveau des vannes **17**, **18** ou un mauvais comptage. Si les coefficients **Ko** et **Ko'** présentent des valeurs identiques, le dispositif **2** valide le coefficient initial d'étalonnage **Ko** qui permettra de corriger les mesures de débit effectuées par les débitmètres lors de la séance de dialyse.

Le procédé selon l'invention autorise l'étalonnage des débitmètres pendant la séance de dialyse qui suit la phase initiale d'étalonnage. Pendant une telle séance de dialyse, il est procédé à au moins une phase de détermination, respectivement **Phei**, **Phsj** d'un nouveau coefficient auxiliaire d'étalonnage pour le capteur d'entrée **De** et pour le capteur de sortie **Ds**.

Tel que cela apparaît plus précisément à la **fig. 3**, la phase de détermination **Phe₁** d'un nouveau coefficient auxiliaire d'étalonnage pour le capteur d'entrée **De**, présentant une durée **Te₁**, consiste à fermer la vanne **17** et à ouvrir la vanne **18** et à commander les vannes **19** et **20** de manière que le liquide de dialyse circule dans la canalisation amont **8** placée en série avec la branche amont de dérivation **13** et dans la canalisation aval **9**. Le capteur auxiliaire **Da** est ainsi placé en série avec le capteur d'entrée **De**. Dans la mesure où les capteurs **De** et **Da** sont parcourus par un même débit de liquide de dialyse, il peut être procédé à un nouvel étalonnage du capteur **De** en comparaison avec le capteur **Da**. A cet effet, les facteurs de réponse **Fe₁** et **Fa₁** des capteurs **De** et **Da** sont à nouveau déterminés par le dispositif **2**, de manière à définir un nouveau coefficient auxiliaire **ke₁**, tel que **ke₁**= **Fe₁**/**Fa₁**.

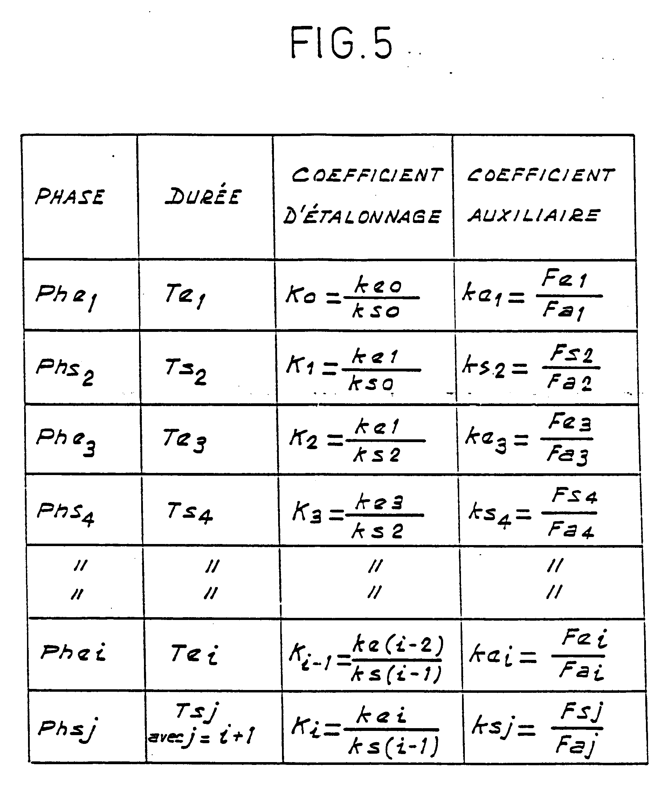

Ce nouveau coefficient **ke₁** est destiné à remplacer, dans le coefficient d'étalonnage **Ko**, le coefficient auxiliaire **keo** du capteur **De**, qui a été défini lors de la phase initiale d'étalonnage. Un nouveau coefficient d'étalonnage **K₁** est défini, tel que **K₁**= **ke₁**/**kso** (**fig. 5**). Il est à noter que le coefficient initial **Ko** est utilisé pendant cette phase **Phe₁** de durée **Te₁**, tandis que le nouveau coefficient **K₁** est pris en compte pour la suite de la séance de dialyse, à savoir la phase **Phs₂**.

La phase **Phe₁** est suivie par une phase **Phs₂** de durée **Ts₂** destinée à assurer la détermination d'un nouveau coefficient auxiliaire d'étalonnage pour le capteur de sortie **Ds**. Comme cela ressort plus précisément de la **fig. 4**, lors de cette phase, la vanne **17** est ouverte et la vanne **18** est fermée, tandis que les vannes **19**, **20** sont commandées de telle sorte que le liquide de dialyse circule dans la branche de dérivation aval **14**. Le liquide de dialyse circule ainsi dans la canalisation amont **8** et la canalisation aval **9** placée en série avec la branche de dérivation **14**. Le capteur auxiliaire **Da** est ainsi placé en série avec le capteur de sortie **Ds**. Il est à noter que la branche de dérivation **14** est raccordée à la canalisation amont **9**, de telle sorte que le liquide de dialyse circule dans la portion commune **15** dans un sens identique au trajet du liquide circulant dans la branche **13**.

Pendant cette phase **Phs₂**, des facteurs de réponse **Fs₂** et **Fa₂**, respectivement pour les capteurs **Ds** et **Da**, sont déterminés par le dispositif **2**, en vue de définir un nouveau coefficient auxiliaire **ks₂** pour le capteur de sortie **Ds**, tel que **ks₂**= **Fs₂**/**Fa₂**. Ce nouveau coefficient **ks₂** est destiné à remplacer, dans le coefficient d'étalonnage **K₁**, le coefficient auxiliaire **kso** du capteur **Ds** qui a été défini lors de la phase initiale d'étalonnage. Un nouveau coefficient d'étalonnage **K₂** est donc défini, avec **K₂**= **ke₁**/**ks₂**.

Avantageusement, les phases de détermination **Phei** et **Phsj** des coefficients auxiliaires **kei**, **ksj** pour les capteurs d'entrée **De** et de sortie **Ds**, sont réalisées, alternativement et successivement, au cours d'une séance de dialyse, de manière à définir des coefficients d'étalonnage **Ki** successifs. Il est à noter que, dans la description qui précède, les coefficients d'étalonnage **Ki** sont modifiés dès la détermination d'un nouveau coefficient auxiliaire **kei** ou **ksj**. Bien entendu, il peut être envisagé de modifier les coefficients d'étalonnage **Ki** uniquement lorsque sont déterminés, à la fois, un nouveau coefficient auxiliaire **kei** et un nouveau coefficient auxiliaire **ksj**.

De préférence, les phases **Phei** et **Phsj** sont réalisées d'une manière consécutive. A titre d'exemple, il peut être envisagé que chaque phase **Phei**, **Phsj** de détermination d'un coefficient auxiliaire, respectivement **kei** et **ksj**, s'effectue pendant une durée **Tei** = **Tsj** = 5 minutes. Ainsi, il peut être obtenu un nouveau coefficient d'étalonnage **Ki** toutes les 5 minutes, si les phases sont réalisées consécutivement les unes aux autres.

Le procédé d'étalonnage selon l'invention peut donc être effectué pendant toute la durée de la séance de dialyse, sans affecter l'efficacité de la séance, dans la mesure où l'étalonnage est réalisé sans mettre en court-circuit l'hémodialyseur. Il peut être procédé ainsi à de nombreux étalonnages au cours de la séance de dialyse, de manière à assurer une bonne précision des mesures tout au long de la séance. Par ailleurs, il est à noter que l'étalonnage du débitmètre d'entrée **De** s'effectue avec du liquide de dialyse frais, tandis que celui du débitmètre de sortie **Ds** est réalisé avec du liquide de dialyse usé, de sorte que les phases d'étalonnage et de mesure s'effectuent dans les mêmes conditions.

Avantageusement, chaque coefficient auxiliaire d'étalonnage **kei, ksj** est déterminé à partir de la moyenne d'une série de coefficients auxiliaires élémentaires déterminés au cours d'une phase de correction correspondante. Dans l'exemple pris ci-dessus, il peut être envisagé de procéder à la moyenne de dix coefficients auxiliaires élémentaires déterminés pendant la phase de correction de durée **Ti** = 5 minutes.

Selon une caractéristique avantageuse du procédé selon l'invention, les coefficients auxiliaires **kei**, **ksj** des capteurs **De** et **Ds** sont comparés, respectivement, aux coefficients auxiliaires correspondants déterminés au cours d'une phase précédente de même nature, de manière à autoriser la détection d'un éventuel défaut apparaisant sur les capteurs **De** ou **Ds**. Bien entendu, un signal d'avertissement est délivré lorsque la différence entre ces valeurs dépasse un seuil donné. Il est à noter que la mise en oeuvre d'un unique débitmètre auxiliaire **Da**, dont les données sont susceptibles d'être transmises à un système de protection indépendant du dispositif de mesure et de contrôle du rein, permet d'obtenir une sécurité optimale sur les valeurs délivrées par les débitmètres.

Par ailleurs, il est à noter que le procédé d'étalonnage selon l'invention peut être mis en oeuvre sur tous types de circuit de dialyse **1** équipant un hémodialyseur. Par exemple, le procédé d'étalonnage selon l'invention peut être appliqué aux débitmètres d'un circuit de liquide de dialyse dont le débit est maintenu constant à l'entrée et à la sortie par la pompe **12**.

Il doit être considéré, également, que le procédé d'étalonnage selon l'invention est avantageusement mis en oeuvre par des moyens de programmation implantés à l'intérieur du dispositif de mesure et de commande **2**.

## Revendications

1. Procédé pour assurer l'étalonnage d'un couple de capteurs (**De**, **Ds**) dont l'un est monté à l'entrée et l'autre à la sortie d'un circuit de dialyse (**1**) destiné à être relié à un hémodialyseur (**4**), le procédé consistant :
- à effectuer une phase initiale d'étalonnage des capteurs (**De**, **Ds**) pendant laquelle l'hémodialyseur (**4**) est mis en court-circuit, de manière à déterminer le facteur de réponse (**Feo**, **Fso**) de chaque capteur (**De**, **Ds**) et à définir un coefficient initial d'étalonnage (**Ko**) permettant d'obtenir une valeur identique de mesure pour chaque capteur (**De**, **Ds**),
- et à réaliser au moins une phase de correction du coefficient initial d'étalonnage (**Ko**), au cours d'une séance de fonctionnement de l'hémodialyseur (**4**),
caractérisé en ce qu'il consiste :
- à réaliser au moins une phase de détermination (**Phei**) d'un coefficient auxiliaire (**kei**) d'étalonnage pour le capteur d'entrée (**De**) consistant :
. à assurer la mise en série, avec le capteur d'entrée (**De**), d'un capteur auxiliaire (**Da**) de même nature que les capteurs d'entrée (**De**) et de sortie (**Ds**),
. à définir les facteurs de réponse (**Fei**, **Fai**) pour les capteurs d'entrée (**De**) et auxiliaire (**Da**),
. et à définir le coefficient auxiliaire d'étalonnage (**kei**) pour le capteur d'entrée (**De**), permettant d'obtenir une même valeur de mesure pour les capteurs d'entrée (**De**) et auxiliaire (**Da**),
- à réaliser au moins une phase de détermination (**Phsj**) d'un coefficient auxiliaire d'étalonnage (**ksj**) pour le capteur de sortie (**Ds**), consistant :
. à assurer la mise en série du capteur de sortie (**Ds**) avec le capteur auxiliaire (**Da**),
. à définir les facteurs de réponse (**Fsj**, **Faj**) pour les capteurs de sortie (**Ds**) et auxiliaire (**Da**),
. et à définir le coefficient d'étalonnage auxiliaire (**ksj**) pour le capteur de sortie (**Ds**), permettant d'obtenir une même valeur de mesure pour les capteurs de sortie (**Ds**) et auxiliaire (**Da**),
- et à déterminer, à partir d'au moins un coefficient auxiliaire d'étalonnage, un nouveau coefficient d'étalonnage (**Ki**) pour les deux capteurs (**Ds**, **De**).

2. Procédé selon la revendication 1, caractérisé en ce qu'il consiste à réaliser, successivement et alternativement, les phases (**Phei**, **Phsj**) de détermination des coefficients auxiliaires (**kei**, **ksj**) pour les capteurs d'entrée (**De**) et de sortie (**Ds**).

3. Procédé selon la revendication 1 ou 2, caractérisé en ce qu'il consiste à réaliser, consécutivement et alternativement, les phases (**Phei**, **Phsj**) de détermination des coefficients auxiliaires d'étalonnage (**kei**, **ksi**) pour les capteurs d'entrée (**De**) et de sortie (**Ds**).

4. Procédé selon la revendication 2 ou 3, caractérisé en ce qu'il consiste à remplacer, après chaque phase (**Phei**, **Phsj**), par le coefficient auxiliaire (**kei** ou **ksj**), dans le coefficient d'étalonnage (**Ki**), le coefficient auxiliaire correspondant défini lors d'une phase précédente.

5. Procédé selon la revendication 1 ou 2, caractérisé en ce qu'il consiste à déterminer chaque coefficient auxiliaire (**kei**, **ksj**) d'un capteur, à partir de la moyenne d'une série de coefficients auxiliaires élémentaires déterminés au cours d'une phase correspondante.

6. Procédé selon la revendication 1, caractérisé en ce qu'il consiste à effectuer la phase initiale d'étalonnage en mettant en série les capteurs d'entrée (**De**), de sortie (**Ds**) et auxiliaire (**Da**), de manière à définir, entre les capteurs d'entrée (**De**) et auxiliaire (**Da**), d'une part, et entre les capteurs de sortie (**Ds**) et auxiliaire (**Da**), d'autre part, respectivement, des coefficients auxiliaires d'étalonnage (keo, kso) destinés à former le coefficient initial d'étalonnage (**Ko**).

7. Procédé selon la revendication 6, caractérisé en ce qu'il consiste, lors de la phase initiale d'étalonnage, à définir un coefficient (**Ko'**) correspondant au rapport entre les facteurs de réponse (**Feo** et **Fso**) des capteurs (**De** et **Ds**), à comparer les coefficients (**Ko′** et **Ko**) et à valider le coefficient initial d'étalonnage (**Ko**) uniquement si les valeurs des deux coefficients (**Ko** et **Ko′**) sont identiques.

8. Procédé selon la revendication 1, caractérisé en ce qu'il consiste à définir le coefficient d'étalonnage (**Ki**) en procédant au rapport des coefficients auxiliaires d'étalonnage du capteur d'entrée (**kei**) et du capteur de sortie (**ksj**).

9. Procédé selon la revendication 1, caractérisé en ce qu'il consiste à comparer, lors de chacune des phases de détermination (**Phei**, **Phsj**), les coefficients auxiliaires (**kei**, **ksj**) des capteurs d'entrée et de sortie (**De**, **Ds**) aux coefficients auxiliaires correspondants déterminés au cours d'une phase précédente de même nature et à délivrer un signal d'avertissement lorsque la différence entre les coefficients auxiliaires dépasse un seuil donné.

10. Procédé selon la revendication 1, caractérisé en ce qu'il consiste, lors des phases de détermination (**Phei**, **Phsj**), à assurer la mise en série du capteur auxiliaire (**Da**) avec le capteur d'entrée (**De**) ou le capteur de sortie (**Ds**), de manière que le liquide de dialyse circule toujours dans le même sens à travers le capteur auxiliaire (**Da**).

11. Rein artificiel pour la mise en oeuvre du procédé selon les revendications 1 à 10, comprenant :
- un circuit de liquide de dialyse (**1**) comportant, d'une part, une canalisation d'entrée (**8**) équipée d'au moins un capteur (**De**) et destinée à être reliée à un premier compartiment (**3**) d'un hémodialyseur (**4**) et, d'autre part, une canalisation de sortie (**9**) équipée d'au moins un capteur (**Ds**) et destinée à être reliée à la sortie du premier compartiment (**3**) qui est séparé par une membrane semi-perméable (**6**), d'un second compartiment (**5**) destiné à être relié à un circuit (**7**) pour la circulation extra-corporelle de sang,
- des moyens (**11**, **12**) pour déplacer le liquide de dialyse,
- et un dispositif (**2**) de mesure et de commande, relié aux capteurs (**De**, **Ds**) et permettant de définir un coefficient d'étalonnage pour les capteurs (**De**, **Ds**),
caractérisé en ce que :
- le circuit de liquide de dialyse (**1**) comporte :
. une branche amont (**13**) de dérivation à la canalisation d'entrée (**8**) et une branche aval (**14**) de dérivation à la canalisation de sortie (**9**), les branches de dérivation ayant une portion commune (**15**) dans laquelle est monté au moins un capteur auxiliaire (**Da**) de même nature que les capteurs d'entrée (**De**) et de sortie (**Ds**),
. et des moyens d'obturation (**17**, **18**, **19**, **20**) pilotés par le dispositif de commande (**2**) et permettant la circulation du liquide de dialyse, soit dans l'une, soit dans l'autre branche de dérivation,
- et en ce que le dispositif de commande (**2**) comporte :
. des moyens pour déterminer la durée (**Tei**, **Tej**) et la fréquence des phases de correction (**Phei**, **Phsj**) des coefficients d'étalonnage (**Ki**) des capteurs d'entrée (**De**) et de sortie (**Ds**),
. des moyens pour définir, pendant chaque phase de correction, des coefficients auxiliaires d'étalonnage (**kei**, **ksj**) pour les capteurs d'entrée (**De**) et de sortie (**Ds**), ces coefficients correspondant, respectivement, à une relation entre les capteurs auxiliaire (**Da**) et d'entrée (**De**) et entre les capteurs auxiliaire (**Da**) et de sortie (**Ds**),
. et des moyens pour corriger le coefficient d'étalonnage (**Ki**) par au moins un coefficient auxiliaire d'entrée (**kei**) ou de sortie (**ksj**) précédemment calculés.

## Patentansprüche

1. Verfahren zum Sicherstellen der Eichung eines Paars von Meßfühlern (De, Ds), von denen einer am Einlaß und der andere am Auslaß eines Dialysekreislaufs (1) angeordnet ist, der dazu bestimmt ist, mit einem Blutdialysator (4) verbunden zu werden, wobei das Verfahren umfaßt:
- Bewirken einer Anfangseichphase für die Meßfühler (De, Ds), während welcher der Blutdialysator (4) kurzgeschlossen ist, um den Ansprechfaktor (Feo, Fso) eines jeden Meßfühlers (De, Ds) zu bestimmen, und um einen Anfangseichkoeffizienten (Ko) festzulegen, der es erlaubt, einen identischen Meßwert für jeden Meßfühler (De, Ds) zu erhalten,
- Realisieren zumindest einer Korrekturphase für den Anfangseichkoeffizienten (Ko) im Laufe einer Arbeitssitzung des Blutdialysators (4),
dadurch gekennzeichnet, daß es umfaßt:
- Realisieren zumindest einer Phase (Phei) zum Bestimmen eines zusätzlichen Eichkoeffizienten (kei) für den Einlaßmeßfühler (De) umfassend :
. Sicherstellen der in Reihe Schaltung eines zusätzlichen Meßfühlers (Da) derselben Art wie der Einlaßmeßfühler (De) und der Auslaßmeßfühler (Ds) mit dem Einlaßmeßfühler (De),
. Festsetzen der Ansprechfaktoren (Fei, Fai) für den Einlaßmeßfühler (De) und den zusätzlichen Meßfühler (Da),
. Festsetzen des zusätzlichen Eichkoeffizienten (Kei) für den Einlaßmeßfühler (De), der das Erhalten eines selben Meßwerts für den Einlaßmeßfühler (De) und den zusätzlichen Meßfühler (Da) erlaubt,
- Realisieren zumindest einer Phase (Phsj) zum Bestimmen eines zusätzlichen Eichkoeffizienten (ksj) für den Auslaßmeßfühler (Ds), umfassend :
. Sicherstellen der in Reihe Schaltung des Auslaßmeßfühlers (Ds) mit dem zusätzlichen Meßfühler (Da),
. Festsetzen der Ansprechfaktoren (Fsj, Faj) für den Auslaßmeßfühler (Ds) und den zusätzlichen Meßfühler (Da),
. Festsetzen des zusätzlichen Eichkoeffizienten (ksj) für den Auslaßmeßfühler (Ds), der das Erhalten eines selben Meßwerts für den Auslaßmeßfühler (Ds) und den zusätzlichen Meßfühler (Da) erlaubt,
- und Festsetzen eines neuen Eichkoeffizienten (Ki) für die beiden Meßfühler (De, Da), ausgehend von zumindest einem zusätzlichen Eichkoeffizienten.

2. Verfahren nach Anspruch 1, dadurch gekennzeichnet, daß es umfaß : aufeinanderfolgendes und abwechselndes Realisieren der Phasen (Phei, Phsj) zum Bestimmen der zusätzlichen Eichkoeffizienten (kei, ksj) für den Einlaßmeßfühler (De) und den Auslaßmeßfühler (Ds).

3. Verfahren nach Anspruch 1 oder 2, dadurch gekennzeichnet, daß es umfaßt : unmittelbar aufeinanderfolgendes und abwechselndes Realisieren der Phasen (Phei, Phsj) zur Bestimmung der zusätzlichen Eichkoeffizienten (kei, ksj) für den Einlaßmeßfühler (De) und den Auslaßmeßfühler (Ds).

4. Verfahren nach Anspruch 2 oder 3, dadurch gekennzeichnet, daß es umfaßt : Ersetzen des während der vorausgehenden Phase festgesetzten entsprechenden zusätzlichen Koeffizienten nach jeder Phase (Phei, Phsj) durch den zusätzlichen Koeffizienten (kei, ksj) im Eichkoeffizienten (Ki).

5. Verfahren nach Anspruch 1 oder 2, dadurch gekennzeichnet, daß es umfaßt : Bestimmen jedes zusätzlichen Koeffizienten (kei, ksj) für einen Meßfühler, ausgehend vom Mittelwert einer Serie von elementaren zusätzlichen Koeffizienten, die im Laufe der entsprechenden Phase bestimmt worden sind.

6. Verfahren nach Anspruch 1, dadurch gekennzeichnet, daß es umfaßt : Bewirken der Anfangseichphase durch in Serie Schalten des Einlaßmeßfühlers (De), des Auslaßmeßfühlers (Ds) und des zusätzlichen Meßfühlers (Da) derart, daß zwischen dem Einlaßmeßfühler (De) und dem zusätzlichen Meßfühler (Da) einerseits und zwischen dem Auslaßmeßfühler (Ds) und dem zusätzlichen Meßfühler (Da) andererseits jeweils die zusätzlichen Eichkoeffizienten (keo, kso) festgesetzt werden, die dazu bestimmt sind, den Anfangseichkoeffizienten (Ko) zu bilden.

7. Verfahren nach Anspruch 6, dadurch gekennzeichnet, daß es während der Anfangseichphase umfaßt : Festsetzen eines Koeffizienten (Ko′) entsprechend dem Verhältnis zwischen den Ansprechfaktoren (Feo und Fso) der Meßfühler (De und Ds), Vergleichen der Koeffizienten (Ko′ und Ko) und Gültigmachen des Anfangseichkoeffizienten (Ko) nur dann, wenn die beiden Koeffizienten (Ko und Ko′) identisch sind.

8. Verfahren nach Anspruch 1, dadurch gekennzeichnet, daß es umfaßt : Festsetzen des Eichkoeffizienten (Ki) durch in Verhältnis setzen der zusätzlichen Eichkoeffizienten des Einlaßmeßfühlers (kei) und des Auslaßmeßfühlers (ksj).

9. Verfahren nach Anspruch 1, dadurch gekennzeichnet, daß es umfaßt : Vergleichen, während jeder der Bestimmungsphasen (Phei, Phsj), der zusätzlichen Koeffizienten (kei, ksj) des Eingangsmeßfühlers (De) und des Auslaßmeßfühlers (Ds) jeweils mit entsprechenden zusätzlichen Koeffizienten, die im Lauf einer vorausgehenden Phase derselben Art bestimmt worden sind, und Abgeben eines Warnsignals, wenn die Differenz zwischen den zusätzlichen Koeffizienten einen Sollwert überschreitet.

10. Verfahren nach Anspruch 1, dadurch gekennzeichnet, daß es während den Bestimmungsphasen (Phei, Phsj) umfaßt : Sicherstellen der in Reihe Schaltung des zusätzlichen Meßfühlers (Da) mit dem Einlaßmeßfühler (De) oder dem Auslaßmeßfühler (Ds), damit die Dialyseflüssigkeit immer in derselben Richtung durch den zusätzlichen Meßfühler (Da) zirkuliert.

11. Künstliche Niere zum Durchführen des Verfahrens gemäß den Ansprüchen 1 bis 10, mit:
- einem Dialyseflüssigkeitskreislauf (1), der einerseits eine Einlaßleitung (8) umfaßt, die zumindest mit einem Meßfühler (De) versehen und dazu bestimmt ist, mit einem ersten Teilraum (3) eines Blutdialysators (4) verbunden zu werden, und andererseits eine Auslaßleitung (9), die mit zumindest einem Meßfühler (Ds) versehen und dazu bestimmt ist, mit dem Auslaß des ersten Teilraums (3) verbunden zu werden, der durch eine halbdurchlässige Membran (6) von einem zweiten Teilraum (5) getrennt ist, der dazu bestimmt ist, mit einem Kreislauf (7) für die Zirkulation extrakorporellen Bluts verbunden zu werden,
- einer Einrichtung (11, 12) zum Bewegen der Dialyseflüssigkeit,
- und eine Meß- und Steuervorrichtung (2), die mit den Meßfühlern (De, Ds) verbunden ist, und es erlaubt, einen Eichkoeffizienten für die Meßfühler (De, Ds) festzusetzen,
dadurch gekennzeichnet, daß
- die Dialyseflüssigkeit (1) umfaßt :
. einen stromaufwärtigen Zweig (13) zum Umleiten zur Einlaßleitung (8) und einen stromabwärtigen Zweig (14) zum Umleiten zur Auslaßleitung (9), wobei die Umleitungszweige einen gemeinsamen Bereich (15) haben, in dem zumindest ein zusätzlicher Meßfühler (Da) derselben Art wie der Einlaßmeßfühler (De) und der Auslaßmeßfühler (Ds) angebracht ist, und
. Absperreinrichtungen (17, 18, 19, 20), die durch die Steuereinrichtung (2) gesteuert sind und die Zirkulation der Dialyseflüssigkeit entweder in einem oder dem anderen Umleitungszweig erlauben,
- und daß die Steuervorrichtung (2) umfaßt:
. eine Einrichtung zum Bestimmen der Dauer (Tei, Tej) und der Frequenz der Korrektorphasen (Phei, Phsj) der Eichkoeffizienten (Ki) des Einlaßmeßfühlers (De) und des Auslaßmeßfühlers (Ds),
. eine Einrichtung zum Festsetzen, während jeder Korrekturphase, zusätzliche Eichkoeffizienten (kei, ksj) für den Einlaßmeßfühler (De) und den Auslaßmeßfühler (Ds), wobei diese Koeffizienten jeweils eine Beziehung zwischen dem zusätzlichen Meßfühler (Da) und dem Einlaßmeßfühler (De) und zwischen dem zusätzlichen Meßfühler (Da) und dem Auslaßmeßfühler (Ds) entsprechen,
. und eine Einrichtung zum Korrigieren des Eichkoeffizienten (Ki) durch zumindest einen zusätzlichen Einlaßkoeffizienten (kei) oder Auslaßkoeffizienten (ksj), die vorausgehend berechnet worden sind.

## Claims

1. A method to ensure the calibration of a pair of sensors (De, Ds), one of which is mounted at the intake and the other at the outlet of a dialysis circuit (1) intended to be connected to a haemodialyser (4), the method lying in :
- carrying out an initial calibration stage of the sensors (De, Ds) during which the haemodialyser (4) is bypassed so as to determine the response factor (Feo, Fso) of each sensor (De, Ds) and in defining an initial calibration coefficient (Ko) making it possible to obtain an identical measurement value for each sensor (De, Ds),
- and in obtaining at least one correction stage of the initial calibration coefficient (Ko) in the course of an operating session of the haemodialyser,
characterized in that it lies in :
- obtaining at least one stage (Phei) for determining an auxiliary calibration coefficient (kei) for the intake sensor (De) constituted by:
. ensuring that an auxiliary sensor (Da) of the same kind as the intake and outlet sensors (De, Ds) is placed in series with the intake sensor (De),
. defining the response factors (Fei, Fai) for the intake and auxiliary sensors (De, Da),
. and defining the auxiliary calibration coefficient (kei) for the intake sensor (De), making it possible to obtain the same measurement value for the intake and auxiliary sensors (De, Da),
- obtaining at least one stage (Phsj) for determining an auxiliary calibration coefficient (ksj) for the outlet sensor (Ds), constituted by:
. ensuring that the outlet sensor (Ds) is placed in series with the auxiliary sensor (Da),
. defining the response factors (Fsj, Faj) for the outlet and auxiliary sensors (Ds, Da),
. and defining the auxiliary calibration coefficient (ksj) for the outlet sensor (Ds), making it possible to obtain the same measurement value for the outlet and auxiliary sensors (Ds, Da),
- and determining on the basis of at least one auxiliary calibration coefficient, a new calibration coefficient (Ki) for the two sensors (De, Ds).

2. Method according to claim 1, characterized in that it lies in obtaining, successively and alternately, the stages (Phei, Phsj) for determining the auxiliary coefficients (kei, ksj) for the intake and outlet sensors (De, Ds).

3. Method according to claim 1 or 2, characterized in that it lies in obtaining, alternately and one following the other, the stages (Phei, Phsj) for determining the auxiliary calibration coefficients (kei, ksj) for the intake and outlet sensors (De, Ds).

4. Method according to claim 2 or 3, characterized in that it lies in replacing, after each stage (Phei, Phsj), the corresponding auxiliary coefficient defined during a preceding stage by the auxiliary coefficient (kei or ksj) in the calibration coefficient (Ki).

5. Method according to claim 1 or 2, characterized in that it lies in determining each auxiliary coefficient (kei, ksj) of a sensor, on the basis of the average of a series of elementary auxiliary coefficients determined in the course of a corresponding stage.

6. Method according to claim 1, characterized in that it lies in effecting the initial calibration stage by placing the intake sensor (De), the outlet sensor (Ds) and the auxiliary sensor (Da) in series so as to define between the intake and auxiliary sensors (De, Da) on the one hand, and the outlet and auxiliary sensors (Ds, Da) on the other hand, auxiliary calibration coefficients (keo, kso) respectively, intended to form the initial calibration coefficient (Ko).

7. Method according to claim 6, characterized in that it lies in defining, during the initial calibration stage, a coefficient (Ko') corresponding to the ratio between the response factors (Feo and Fso) of the sensors (De and Ds), in comparing the coefficients (Ko' and Ko) and in validating the initial calibration coefficient (Ko) only if the values of the two coefficients (Ko and Ko') are identical.

8. Method according to claim 1, characterized in that it lies in defining the calibration coefficient (Ki) by forming the ratio of the auxiliary calibration coefficients of the intake sensor (kei) and of the outlet sensor (ksj).

9. Method according to claim 1, characterized in that it lies in comparing, during each one of the determination stages (Phei, Phsj), the auxiliary coefficients (kei, ksj) of the intake and outlet sensors (De, Ds) with corresponding auxiliary coefficients defined during a previous stage of the same kind, and of delivering a warning signal when the difference between the auxiliary coefficients exceeds a given threshold.

10. Method according to claim 1, characterized in that it lies, during the determination stages (Phei, Phsj), in placing in series the auxiliary sensor (Da) with the intake sensor (De) or the outlet sensor (Ds), so that the dialysis liquid always circulates in the same direction through the auxiliary sensor (Da).

11. An artificial kidney for performing the method according to claims 1 to 10 including:
- a dialysis liquid circuit (1) comprising, on the one hand, an intake line (8) fitted with at least one sensor (De) and intended to be connected to a first compartment (3) of a haemodialyser (4), and, on the other hand, an outlet line (9) fitted with at least one sensor (Ds) and intended to be connected to the outlet of the first compartment (3) which is separated by a semi-permeable membrane (6) from a second compartment (5) intended to be connected to a circuit (7) for the extracorporeal circulation of the blood,
- means (11, 12) for displacing the dialysis liquid,
- and a measurement and control device (2) connected to the sensors (De, Ds) and allowing a calibration coefficient to be defined for the sensors (De, Ds),
characterized in that
- the dialysis liquid circuit (1) includes:
. an upstream branch (13) for bypassing the intake line (8) and a downstream branch (14) for bypassing the outlet line (9), the bypass branches having a common portion (15) wherein there is mounted at least one auxiliary sensor (Da) of the same kind as the intake and outlet sensors (De, Ds),
. and obturating means (17, 18, 19, 20) controlled by the control device (2) and allowing the dialysis liquid to circulate either in the one or in the other bypass branch,
- and in that the control device (2) includes:
. means for determining the duration (Tei, Tej) and the frequency of the correction stages (Phei, Phsj) of the calibration coefficients (Ki) of the intake and outlet sensors (De, Ds),
. means for defining during each correction stage auxiliary calibration coefficients (kei, ksj) for the intake sensor (De) and the outlet sensor (Ds), these coefficients corresponding respectively to a relation between the auxiliary and intake sensors (Da, De) and the auxiliary and outlet sensors (Da, Ds),
. and means for correcting the calibration coefficient (Ki) by at least one auxiliary intake coefficient (kei) or outlet coefficient (ksj) that has been previously calculated.
